(19) Europäisches Patentamt
European Patent Office
Office européen des brevets

(11) Veröffentlichungsnummer: **0 328 999 B1**

(12) **EUROPÄISCHE PATENTSCHRIFT**

(45) Veröffentlichungstag der Patentschrift: **16.12.92**

(51) Int. Cl.5: **C07D 277/42**, C07D 277/36, C07D 277/52, A01N 43/78

(21) Anmeldenummer: **89102090.1**

(22) Anmeldetag: **08.02.89**

Die Akte enthält technische Angaben, die nach dem Eingang der Anmeldung eingereicht wurden und die nicht in dieser Patentschrift enthalten sind.

(54) **N-sulfenylierte 2-Amino-4-chlorthiazol-sulfonamide, ihre Verwendung, Verfahren zu ihrer Herstellung und die dabei auftretenden Zwischenprodukte 2,4-Dichlor-thiazol-sulfonsäurechlorid und 2-Amino-4-chlor-thiazol-sulfonsäureamid.**

(30) Priorität: **18.02.88 DE 3805058**

(43) Veröffentlichungstag der Anmeldung:
**23.08.89 Patentblatt 89/34**

(45) Bekanntmachung des Hinweises auf die Patenterteilung:
**16.12.92 Patentblatt 92/51**

(84) Benannte Vertragsstaaten:
**DE FR GB IT**

(56) Entgegenhaltungen:
**EP-A- 0 276 177**
**EP-A- 0 296 673**
**EP-A- 0 301 613**
**EP-A- 0 328 954**
**DE-A- 1 193 498**

**CHEMICAL ABSTRACTS, Band 78, Nr. 19, 14. Mai 1973, Columbus, Ohio, US; SKOPENKO, V.N. et al.: "Sulfonation and chlorosulfonylation of 6-phenyl-thiazolo[2,3-e]tetrazole" Seite 477, Spalte 2, Zusammenfassung-Nr. 124 507a**

(73) Patentinhaber: **BAYER AG**

**W-5090 Leverkusen 1 Bayerwerk(DE)**

(72) Erfinder: **Beck, Gunther, Dr.**
**Am Mittelberg 19**
**W-5090 Leverkusen 1(DE)**
Erfinder: **Baasner, Bernd, Dr.**
**Hamberger-Strasse 27 d**
**W-5090 Leverkusen 3(DE)**
Erfinder: **Schwamborn, Michael, Dr.**
**Von-Lohe-Strasse 9**
**W-5000 Köln 80(DE)**
Erfinder: **Brandes, Wilhelm, Dr.**
**Eichendorffstrasse 3**
**W-5653 Leichlingen 1(DE)**

**EP 0 328 999 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTSRACTS, Band 66, Nr. 3, 16. Januar 1967, Columbus, Ohio, US; SCHMIDT P. et al.: "A new group of antischistosomal compounds" Seite 1054, Spalte 2, Zusammenfassung-Nr. 10 880n

Chemie der Pflanzenschutz- und Schädlingsbekämpfungsmittel, R. Wegler, Band 4 (1977) 192-195

## Beschreibung

Die vorliegende Erfindung betrifft die neuen N-sulfenylierten 2-Amino-4-chlor-thiazol-sulfonamide, ein Verfahren zu ihrer Herstellung, ihre Verwendung als Pflanzenschutzmittel und die bei der Herstellung auftretenden Zwischenprodukte.

Seit langem ist bekannt, daß N-Trihalogenmethylthio-Verbindungen als Fungizide in Landwirtschaft und Gartenbau verwendet werden können. So werden beispielsweise N-(Trichlormethylthio)-tetrahydrophthalimid (DE-PS 887 506) und N,N-Dimethyl-N'-phenyl-N'-(dichlorfluormethylthio)-sulfamid (DE-PS 11 93 498) im Obst- und Weinbau zur Bekämpfung von Pilzkrankheiten eingesetzt. Weiterhin sind auch N-(Dichlorfluormethylthio)-benzol-sulfonamide, wie beispielsweise N-Dichlorfluormethylthio-N-methyl-3,4-dichlorbenzol- bzw. -3-nitrobenzol-sulfonamid, bekannt (siehe ebenfalls DE-PS 11 93 498); keiner dieser vorbekannten Wirkstoffe ist ein Thiazolderivat.

Aus dem letztgenannten Dokument ist außerdem bekannt, daß in dieser Reihe die N-Dichlorfluormethylthio-Derivate im Vergleich zu den entsprechenden N-Trichlormethylthio-Verbindungen (z.B. Captan) eine bessere Wirkung als Fungizide besitzen, wobei als toxiphore Gruppe der N-Dichlorfluormethylthio-Rest N-S-$CFCl_2$ anzusehen ist (vgl. "Chemie der Pflanzenschutz- und Schädlingsbekämpfungsmittel"; R. Wegler, Band 4 (1977), S. 192-195, insbesondere S. 194 oben).

Ferner sind bestimmte pharmazeutisch wirksame Thiazolderivate bekannt, die jedoch keinen 4-Chlor-Substituenten und keine N-Dichlorhalogenomethylthio-Gruppe tragen (vgl. Chem. Abstr. 66, 1054 (1967) 10880 n und Chem. Abstr. 78, 477 (1977) 124507 a).

Es sind auch verschiedene substituierte, fungizid wirksame Thiazolderivate bekanntgeworden, welche jedoch keine 5-Sulfonamid-Gruppe und ebenfalls keine N-Dichlorhalogenomethylthio-Gruppen tragen (vgl. EP-A-0276177, EP-A-0296673, EP-A-0301613 und EP-A-0328954).

(Die Gruppe N-S-$CFCl_2$ wird nachfolgend als "N-Dichlorfluormethylsulfenyl"-Rest bezeichnet.)

Es wurden die neuen N-sulfenylierten 2-Amino-4-chlor-thiazol-sulfonamide der Formel (I)

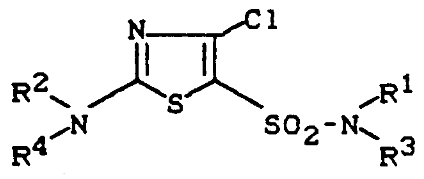

(I),

gefunden, worin

- $R^1$ und $R^2$ unabhängig voneinander geradkettiges oder verzweigtes $C_1$-$C_6$-Alkyl, Allyl, Crotyl, Propargyl, $C_3$-$C_6$-Cycloalkyl, Phenyl, Benzyl, Phenylethyl, 2-Furyl, 2-, 3- oder 4-Pyridyl, 2- oder 4-Pyrimidyl oder 2-Morpholinyl bedeuten und
- $R^3$ und $R^4$ unabhängig voneinander für Wasserstoff, geradkettiges oder verzweigtes $C_1$-$C_6$-Alkyl, $C_3$-$C_6$-Cycloalkyl, Phenyl, Benzyl oder Phenylethyl oder für den Dichlorhalogenomethylsulfenylrest -S-$CCl_2$X, in welchem X Fluor, Chlor oder Brom bedeutet, stehen,

wobei mindestens einer der Reste $R^3$ und $R^4$ für den Rest -S-$CCl_2$X steht.

Es wurde weiterhin ein Verfahren zur Herstellung der Stoffe der Formel (I) gefunden, das dadurch gekennzeichnet ist, daß man in einer ersten Stufe 2,4-Dichlor-thiazol mit Chlorsulfonsäure zu 2,4-Dichlor-thiazol-sulfonsäurechlorid der Formel (II)

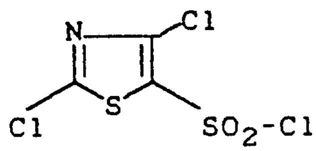

(II),

umsetzt, in einer zweiten Stufe das 2,4-Dichlor-thiazol-sulfonsäurechlorid (II) mit Aminen der Formeln (III) oder (IV)

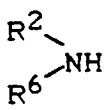 (III) bzw. 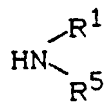 (IV),

in welchen

$R^1$ und $R^2$ die oben angegebene Bedeutung haben und

$R^5$ und $R^6$ unabhängig voneinander für Wasserstoff, $C_1$-$C_6$-Alkyl, $C_3$-$C_6$-Cycloalkyl, Phenyl oder Benzyl stehen,

wobei mindestens einer der Reste $R^5$ und $R^6$ Wasserstoff bedeutet,
zu 2-Amino-4-chlor-thiazol-sulfonamiden der Formel (V)

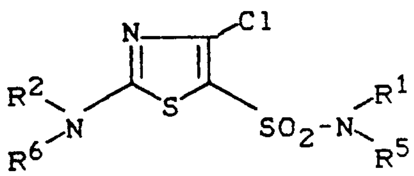 (V)

umsetzt, worin

$R^1$, $R^2$, $R^5$ und $R^6$ die angegebene Bedeutung haben,

und in einer dritten Stufe die 2-Amino-4-chlor-thiazol-sulfonamide (V) mit Dichlorhalogenomethyl-sulfenylchloriden der Formel (VI)

Cl-S-$CCl_2$X (VI),

worin

X Fluor, Chlor oder Brom bedeutet,

zu den Stoffen der Formel (I) umsetzt.

Die Erfindung betrifft weiterhin die neuen Zwischenprodukte der Formeln (II) und (V).

Beispiele für $C_1$-$C_6$-Alkyl sind Methyl, Ethyl, Propyl, Iso-propyl, Butyl, Isobutyl, tert.-Butyl, geradkettiges oder verzweigtes Pentyl oder Hexyl; bevorzugt ist $C_1$-$C_5$-Alkyl.

Beispiele für $C_3$-$C_6$-Cycloalkyl sind Cyclopropyl, Cyclobutyl, Cyclopentyl oder Cyclohexyl.

Die erfindungsgemäßen Stoffe der Formel (I) sind alle gekennzeichnet durch mindestens eine stickstoffständige Dichlorhalogenomethylthio-Gruppe in Verbindung mit einem 2-Amino-4-chlor-5-sulfonamido-thiazol-Gerüst. Unter die Formel (I) fallen demnach folgende Untergruppen:

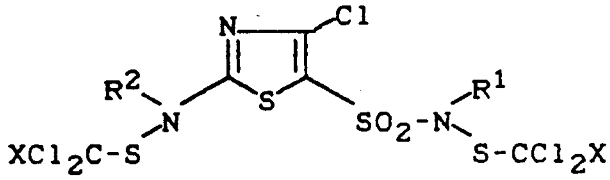

(Ia),

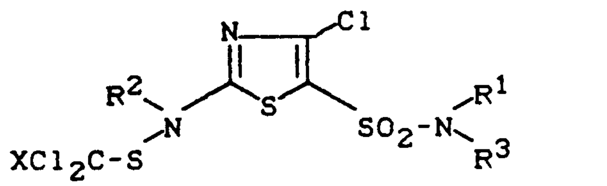

(Ib) und

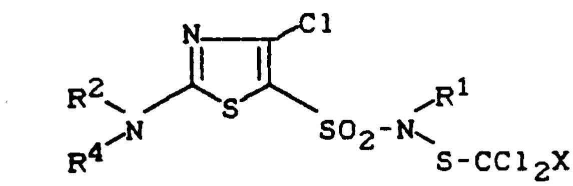

(Ic),

worin
$R^1$, $R^2$, $R^3$, $R^4$ und X die oben angegebene Bedeutung besitzen.

Besonders bevorzugte N-sulfenylierte 2-Amino-4-chlor-thiazol-sulfonamide sind solche der Formel (I)

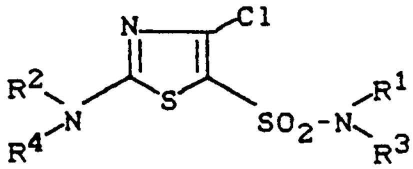

(I),

in der

$R^1$ und $R^2$ unabhängig voneinander geradkettiges oder verzweigtes $C_1$-$C_6$-Alkyl, Allyl, Crotyl, Propargyl, $C_3$-$C_6$-Cycloalkyl, Phenyl oder Benzyl bedeuten und

$R^3$ und $R^4$ unabhängig voneinander für Wasserstoff, geradkettiges oder verzweigtes $C_1$-$C_6$-Alkyl oder den Dichlorhalogenomethylsulfenylrest -S-$CCl_2$X, in welchem X Fluor, Chlor oder Brom bedeutet, stehen,

wobei mindestens einer der Reste $R^3$ und $R^4$ für den Rest -S-$CCl_2$X steht.

Zu ihrer Herstellung werden Amine (III) bzw. (IV) und Stoffe (V) mit entsprechendem Bedeutungsumfang eingesetzt.

Neben den in den Ausführungsbeispielen genannten Verbindungen der Formel (I) sei zusätzlich auf folgende Einzelverbindungen hingewiesen:

| Nr. | $R^1$ | $R^3$ | $R^2$ | $R^4$ |
|---|---|---|---|---|
| Ia.1 | $CH_3$ | $SCFCl_2$ | $CH_3$ | $SCFCl_2$ |
| Ic.1 | $CH_3$ | $SCFCl_2$ | $CH_3$ | $CH_3$ |
| Ic.2 | $(CH_3)_2CH$ | $SCFCl_2$ | $CH_3$ | $CH_3$ |
| Ib.1 | $CH_3$ | $CH_3$ | $(CH_3)_2CH$ | $SCFCl_2$ |
| Ib.2 | $CH_3$ | $CH_3$ | $C_6H_5$ | $SCFCl_2$ |
| Ia.2 | $C_2H_5$ | $SCFCl_2$ | $C_2H_5$ | $SCFCl_2$ |
| Ib.3 | $CH_3$ | $CH_3$ | $CH_3$ | $SCFCl_2$ |
| Ia.3 | $CH_3$ | $SCFCl_2$ | $(CH_3)_2CH$ | $SCFCl_2$ |
| Ia.4 | $(CH_3)_2CH$ | $SCFCl_2$ | $CH_3$ | $SCFCl_2$ |

| Nr. | $R^1$ | $R^3$ | $R^2$ | $R^4$ |
|---|---|---|---|---|
| Ic.3 | $CH_3$ | $SCFCl_2$ | $C_6H_5$ | H |
| Ia.5 | $(CH_3)_3C-CH_2-$ | $SCFCl_2$ | $(CH_3)_3C-CH_2-$ | $SCFCl_2$ |
| 1b.4 | Cyclopropyl | $SCFCl_2$ | Cyclopropyl | $SCFCl_2$ |
| 1c.4 | Cyclopropyl | $SCFCl_2$ | $CH_3$ | $CH_3$ |
| 1c.5 | $CH_2=CH-CH_2$ | $SCFCl_2$ | $CH_3$ | $CH_3$ |
| 1c.6 | $CH\equiv C-CH_2$ | $SCFCl_2$ | $CH_3$ | $CH_3$ |

Zur Herstellung der Stoffe gemäß Formel (I) wird in einer ersten Stufe 2,4-Dichlor-thiazol mit 2-20 Mol, bevorzugt 5-15 Mol Chlorsulfonsäure bei einer Temperatur in der Nähe des Siedepunkts des Reaktionsgemisches umgesetzt. Die überschüssige Chlorsulfonsäure dient gleichzeitig als Lösungsmittel.

In einer zweiten Stufe des Herstellungsverfahrens für Stoffe der Formel (I) wird das 2,4-Dichlor-thiazol-sulfonsäurechlorid mit Aminen der Formeln (III) und/oder (IV) zu 2-Amino-4-chlor-thiazol-sulfonamiden der Formel (V) umgesetzt.

Diese Umsetzung wird mit primären Aminen bzw. einem primären und einem sekundären Amin durchgeführt, so daß am entstandenen Stoff der Formel (V) mindestens ein N-ständiges H-Atom verbleibt. Es wurde gefunden, daß die Sulfonsäurechlorid-Gruppe vor dem 2-ständigen Chloratom reagiert, während

unter den erfindungsgemäßen Bedingungen das 4-ständige Chloratom nicht reagiert. Die zweite Stufe des erfindungsgemäßen Verfahrens läßt sich demnach in mehreren Varianten durchführen, die die große Vielfalt der Stoffe nach Formeln (V) und (I) herzustellen erlaubt:

- In einer ersten Variante wird das Sulfonsäurechlorid zunächst mit einem primären Amin und dann mit einem sekundären Amin umgesetzt, wobei ein N-ständiges H-Atom in der Sulfonamidgruppe entsteht.
- In einer zweiten Variante wird das Sulfonsäurechlorid zunächst mit einem sekundären Amin und dann mit einem primären Amin umgesetzt, wobei ein N-ständiges H-Atom in der 2-ständigen Aminogruppe entsteht.
- In einer dritten Variante wird das Sulfonsäurechlorid zunächst mit einem ersten primären Amin und dann mit einem zweiten primären Amin umgesetzt, wobei an jedem N-Atom ein H-Atom zu stehen kommt und wobei weiterhin beide N-Atome mit unterschiedlichen organischen Substituenten ausgestattet werden können.
- In einer vierten Variante schließlich werden simultan sowohl die Sulfonylchlorid-Gruppe als auch das 2-ständige Cl-Atom durch das gleiche primäre Amin umgesetzt, wobei wiederum an beiden N-Atomen ein H-Atom zu stehen kommt und wobei beide N-Atome mit dem gleichen Substituenten ausgestattet sind.

Pro Mol umzusetzendes Cl-Atom im 2,4-Dichlorthiazol-sulfonsäurechlorid wird 1-1,5 Mol, bevorzugt 1-1,1 Mol, besonders bevorzugt 1-1,05 Mol eines Amins der Formeln (III) bzw. (IV) eingesetzt. Die Temperatur zur Umsetzung der Sulfonsäurechlorid-Gruppe liegt tiefer als die Temperatur zur Umsetzung des 2-ständigen Cl-Atoms. Bei vorsichtigem Arbeiten kann jedoch die Temperatur für die Umsetzung der Sulfonsäurechlorid-Gruppe geringfügig in den Bereich zur Umsetzung des 2-ständigen Cl-Atoms hineinreichen. Als vorsichtiges Arbeiten gilt hierbei beispielsweise die langsame, tropfenweise Zugabe des für die Umsetzung mit der Sulfonsäurechlorid-Gruppe gewählten Amins, die Vermeidung bzw. Zulassung nur eines geringen Überschusses an Amin oder eine größere Verdünnung des Stoffes (II). Der tiefere Temperaturbereich liegt beispielsweise bei -80 ° C bis + 20 ° C, bevorzugt -60 ° C bis 0 ° C, besonders bevorzugt -40 ° C bis -20 ° C. Der höhere Temperaturbereich liegt im allgemeinen bei 0-100 ° C, bevorzugt bei 0-50 ° C. Für den Fall, daß die Sulfonsäurechlorid-Gruppe und das 2-ständige Cl-Atom mit dem gleichen primären Amin umgesetzt werden, braucht die tiefe Temperatur nicht angewandt zu werden; es wird hierbei sofort im Bereich der höheren Temperatur gearbeitet, Für den Fall, daß im Bereich der tieferen Temperatur auch bei vorsichtiger Arbeitsweise bereits geringe Mengen (bis ca. 5 %) des 2-ständigen Chlors reagieren, kann solches unerwünschte Reaktionsprodukt nach vorheriger Erkennung, beispielsweise durch Dünnschichtchromatographie, durch geeignete Reinigungsmethoden, beispielsweise fraktionierte Kristallisation oder geeignete Chromatographie, abgetrennt werden.

Die verschiedenen Varianten der zweiten Stufe des erfindungsgemäßen Verfahrens werden in Gegenwart einer Base als säurebindendes Mittel und gegebenenfalls in Gegenwart eines Verdünnungsmittels durchgeführt. Ein geeignetes Verdünnungsmittel kann beispielsweise zur Einstellung der gewünschten Temperatur im tiefen Temperaturbereich bei gleichzeitiger Aufrechterhaltung der flüssigen Phase wichtig sein.

Als säurebindende Mittel können anorganische Basen, wie Natriumhydroxid, Natriumcarbonat und andere oder tertiäre Amine, wie Pyridin, Triethylamin oder andere, verwendet werden. Solche säurebindende Mittel sind dem Fachmann in ihrer Art bekannt. Die säurebindenden Mittel werden in äquimolarer Menge zu den eingesetzten primären bzw. sekundären Aminen angewandt. Als säurebindendes Mittel kann jedoch auch das umzusetzende Amin selbst benutzt werden, welches dann in der doppelten molaren Menge, bezogen auf das umzusetzende Cl-Atom nach obiger Angabe, eingesetzt wird. Diese Variante kommt insbesondere bei der Umsetzung des 2-ständigen Cl-Atoms oder bei der Umsetzung der Sulfonsäurechlorid-Gruppe und des 2-ständigen Cl-Atoms mit dem gleichen primären Amin in Frage.

Als Verdünnungsmittel kommen alle in bezug auf die Reaktion inerten Lösungsmittel in Frage. Hierzu gehören beispielsweise Kohlenwasserstoffe, wie Toluol, Hexan, Cyclohexan und andere, Halogenkohlenwasserstoffe, wie Methylenchlorid, Chlorbenzol und andere und Ether, wie Dioxan, Tetrahydrofuran, Diethylether und andere, sowie Wasser. Bei Mitverwendung von Wasser kann sowohl in homogener Phase als auch in heterogener Phase gearbeitet werden. Ein teilweises Ausfrieren von Wasser wurde auch bei tiefen Temperaturen nicht beobachtet und würde im übrigen das erfindungsgemäße Verfahren nicht stören. Ihre Menge beträgt, ohne kritisch zu sein, beispielsweise 0,1 - 15 l, bevorzugt 0,2 - 6 l Verdünnungsmittel pro Mol umzusetzenden Stoff (II). Es entspricht ökonomischem und ökologischem Handeln, eine möglichst geringe Menge an Verdünnungsmittel einzusetzen; solche Mengen können durch einfache Vorversuche ermittelt werden,

Von den in der zweiten Stufe des erfindungsgemäßen Verfahrens entstehenden 2-Amino-4-chlor-thiazol-sulfonamiden der Formel (V) sei neben den Ausführungsbeispielen auf die folgenden, durch ihre Substituenten gekennzeichneten Stoffe, beispielhaft hingewiesen:

| Nr. | $R^1$ | $R^5$ | $R^2$ | $R^6$ |
|---|---|---|---|---|
| V.1 | $CH_3$ | H | $CH_3$ | H |
| V.2 | $CH_3$ | H | $CH_3$ | $CH_3$ |
| V.3 | $CH_3$ | $CH_3$ | $CH_3$ | H |
| V.4 | $CH_3$ | $CH_3$ | $\begin{matrix}CH_3\\CH_3\end{matrix}\rangle CH$ | H |
| V.5 | $CH_3$ | $CH_3$ | $C_6H_5$ | H |
| V.6 | $C_2H_5$ | H | $C_2H_5$ | H |
| V.7 | $(CH_3)_3C-CH_2-$ | H | $(CH_3)_3C-CH_2-$ | H |
| V.8 | $CH_3$ | H | $\begin{matrix}CH_3\\CH_3\end{matrix}\rangle CH$ | H |

| Nr. | $R^1$ | $R^5$ | $R^2$ | $R^6$ |
|---|---|---|---|---|
| V. 9 | $\begin{matrix}CH_3\\CH_3\end{matrix}\rangle CH$ | H | $CH_3$ | H |
| V. 10 | $CH_3$ | H | $C_6H_5$ | H |
| V.11 | Cyclopropyl | H | Cyclopropyl | H |
| V.12 | Cyclopropyl | H | $CH_3$ | $CH_3$ |
| V.13 | $CH_2=CH-CH_2$ | H | $CH_3$ | $CH_3$ |
| V.14 | $CH\equiv C-CH_2$ | H | $CH_3$ | $CH_3$ |

In der dritten Stufe des erfindungsgemäßen Verfahrens werden die 2-Amino-4-chlor-thiazol-sulfonamide der Formel (V) mit dem obengenannten Dichlorhalogenomethylsulfenylchlorid der Formel (VI) zu den Endprodukten der Formel (I) umgesetzt. Das Halogen X in der Dichlorhalogenomethyl-Gruppe ist Fluor, Chlor oder Brom, bevorzugt Fluor oder Chlor, besonders bevorzugt Fluor. Solche Dichlorhalogenomethylsulfenylchloride sind dem Fachmann bekannt (Angew. Chem. 76 (1964), 807).

Das Dichlorhalogenomethyl-sulfenylchlorid wird in einer Menge von 0,9 - 1,2, bevorzugt 0,95 - 1,1, besonders bevorzugt 1 - 1,05 Mol pro Äquivalent umzusetzende H-Atome eingesetzt.

Hierbei wird bei Temperaturen im Bereich von 0-100 °C, bevorzugt 20-50 °C, in Gegenwart von säurebindenden Mitteln der oben für die zweite Verfahrensstufe genannten Art gearbeitet. Als Verdünnungsmittel für die dritte Stufe des erfindungsgemäßen Verfahrens kommen die oben für die zweite Stufe bereits genannten Lösungsmittel in den angegebenen Mengen in Frage.

Zur Durchführung dieser dritten Stufe arbeitet man beispielsweise wie folgt; Eine Verbindung der Formel (V) und ein Sulfenylchlorid der Formel (VI) in der genannten Menge werden in einem der genannten Verdünnungsmittel beispielsweise bei Raumtemperatur vorgelegt. Dann wird das säurebindende Mittel portionsweise zugegeben, so daß die dabei ansteigende Reaktionstemperatur im angegebenen Bereich bleibt. Nach Beendigung der Umsetzung wird das N-sulfenylierte 2-Amino-4-chlor-thiazol-sulfonamid mit wasser ausgefällt und in bekannter weise isoliert (beispielsweise durch Filtration oder Zentrifugieren) und gereinigt (beispielsweise durch Umkristallisieren oder chromatographische Methode).

Beim Einsatz von 2 Mol Sulfenylchlorid (VI) pro Mol des Stoffes (V), falls (V) sowohl in der Sulfonamid- als auch in der Aminogruppe Wasserstoff trägt, kommt man zu N-sulfenylierten 2-Amino-4-chlor-thiazol-sulfonamiden der Untergruppe (Ia). Beim Einsatz von Mengen an (VI) unterhalb von 2 Mol pro Mol (V) erhält man Gemische von (Ia), (Ib) und (Ic); auch beim Einsatz von etwa 1 Mol (VI) pro 1 Mol (V) erhält man Gemische von (Ib) und (Ic), gegebenenfalls mit geringen Anteilen an (Ia). Solche Gemische können aufgetrennt werden, beispielsweise durch Kristallisation oder Chromatographie, häufig günstiger durch Chromatographie. Sollten Anteile des aufgetrennten Gemisches nicht verwendet werden, können sie sauer verseift werden (beispielsweise mit HC1 oder $H_2SO_4$ verschiedener Konzentrationen); hierbei wird die -S-$CCl_2$X-Gruppe abgespalten und der verbleibende Stoff (V) steht als wertvolles Zwischenprodukt für die dritte Stufe des erfindungsgemäßen Verfahrens erneut zur Verfügung.

In vielen Fällen ist die Sulfonamidgruppe in (V) reaktiver als die Aminogruppe, so daß eindeutig die Untergruppe (Ic) entsteht, in der die Aminogruppe Wasserstoff trägt.

In den Fällen, bei denen die Sulfonamidgruppe durch $R^1$ oder $R^3$ desaktiviert ist, tritt die Umsetzung vorwiegend an der Aminogruppe ein, wobei Wasserstoff an der Sulfonamidgruppe bestehen bleibt.

Die N-sulfenylierten 2-Amino-4-chlor-thiazol-sulfonamide der Formel (I) weisen eine gute fungizide Wirksamkeit im Pflanzenschutz bei guter Pflanzenverträglichkeit auf. Diese fungizide Wirksamkeit ist überraschenderweise höher als die der aus dem Stand der Technik bekannten Verbindungen, die strukturelle und wirkungsmäßig sehr naheliegende Verbindungen sind. Ihre besondere Wirksamkeit richtet sich gegen der Erreger des Apfelschorfs und gegen die Grauschimmelfäule.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und die Behandlung des Bodens.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, beispielsweise durch Vermischen der Wirkstoffe mit Streckmitteln, z.B. flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können beispielsweise auch organische Lösungsmittel als Hilflösungsmittel angewandt werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, beispielsweise Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid sowie Wasser. Als verflüssigte gasförmige Streckmittel oder Trägerstoffe sind solche Flüssigkeiten gemeint, welche bei normaler Temperatur und unter Normaldruck gasförmig sind, beispielsweise Aerosol-Treibgase, wie niedere Halogenkohlenwasserstoffe, sowie Butan, Propan, Stickstoff und Kohlendioxid. Als feste Trägerstoffe kommen beispielsweise in Frage: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid oder Silikate. Als feste Trägerstoffe für Granulate seien weiterhin beispielsweise genannt: gebrochene und fraktionierte natürliche Gesteine, wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben oder Tabakstengel. als Emulgier- und/oder schaumerzeugende Mittel kommen beispielsweise in Frage: nicht-ionogene bzw. anionische Emulgatoren, wie Polyoxyethylen-Fettsäureester, Polyoxyethylen-Fettalkoholether, beispielsweise Alkylarylpolyglykol-Ether, Alkylsulfonate, Alkylsulfate, Arylsulfonate sowie Eiweißhydrolysate. Als Dispergiermittel kommen beispielsweise in Frage: Lignin-Sulfitablaugen und Methylcellulose.

In den Formulierungen können Haftmittel, wie Carboxymethylcellulose, natürliche uns synthetische pulvrige, körnige oder latexförmige Polymers verwendet werden, wie Gummiarabicum, Polyvinylalkohol oder Polyvinylacetat.

Weiterhin können Farbstoffe, wie anorganische Pigmente, beispielsweise Eisenoxid, Titanoxid, Ferrocyanblau und organische Farbstoffe, wie Alizarin-, Azo- und Metallphthalocyaninfarbstoffe, sowie weiterhin Spurennährstoffe, wie Salze von Eisen, Mangan, Bor, Kupfer, Kobalt, Molybdän und/oder Zink, verwendet werden.

Die Formulierungen enthalten im allgemeinen 0,1-95 Gew.-% Wirkstoff, vorzugsweise 0,5-90 %. Die erfindungsgemäßen Wirkstoffe können in den Formulierungen in Mischung mit anderen bekannten Wirkstoffen vorliegen, wie mit Fungiziden, Insetkiziden, Akariziden und Herbiziden, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertigen Lösungen, emulgierbaren Konzentraten, Emulsionen, Schäumen, Suspensionen, Spritzpulvern, Pasten, löslichen Pulvern, Stäubemitteln und/oder Granulaten, angewendet werden. Die Anwendung geschieht in üblicher Weise, beispielsweise durch Gießen, Verspritzen, Versprühen, Verstreuen, Verstäuben, Verschäumen, Bestreichen usw. Es ist ferner möglich, die Wirkstoffe nach dem Ultra-Low-Volume (ULV)-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größen Bereich variiert werden. Sie liegen im allgemeinen bei 1-0,0001 Gew.-%, bevorzugt bei 0,5-0,001 Gew.-%.

Bei der Saatgutbehandlung werden im allgemeinen Wirkstoffmengen von 0,001-50 g/kg Saatgut, bevorzugt 0,01-10 g benötigt.

Bei der Behandlung des Bodens sind Wirkstoffkonzentrationen von 0,0001-0,1 Gew.-%, bevorzugt 0,0001-0,02 %, am Wirkungsort erforderlich.

Die Erfindung betrifft daher weiterhin die Verwendung der N-sulfenylierten 2-Amino-4-chlor-thiazolsulfonamide der Formel (I) im Pflanzenschutz, bevorzugt als Fungizide.

## Herstellungsbeispiele

### Beispiel 1

Herstellung des 2,4-Dichlorthiazol-sulfonsäurechlorids

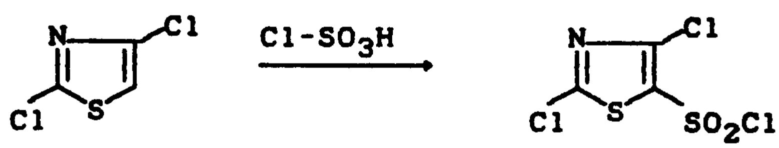


In 2000 ml (30 Mol) Chlorsulfonsäure wurden unter Rühren portionsweise innerhalb von 1,5 Stunden 400 g (2,6 Mol) 2,4-Dichlorthiazol eingetragen, wobei die Temperatur bis etwa 50 ° C stieg. Anschließend wurde etwa 7 Stunden unter Rückfluß zum Sieden erhitzt (Innentemperatur ca. 154 ° C). Nach dem Erkalten wurde tropfenweise auf 10 kg von außen gekühltes und gerührtes Eis/Wasser-Gemisch ausgetragen. Danach wurde der entstandene Niederschlag abfiltriert, mit Wasser neutral gewaschen und bei Raumtemperatur getrocknet. Ausbeute: 357 g (54,4 % der theoretischen Ausbeute).

Die Verbindung kann z.B. aus Petrolether oder aus Acetonitril umkristallisiert oder bei 90 ° /0,1 mbar sublimiert werden. Schmelzpunkt: 60-61 ° C.

### Beispiel 2

Herstellung von 2,4-Dichlor-thiazol-sulfonsäure-N-methylamid

Zu 25,25 g (0,1 Mol) 2,4-Dichlor-thiazol-sulfonsäurechlorid aus Beispiel 1 in 250 ml THF wurden bei ca. -75 ° C in 45 Minuten 21 g einer 30 %igen wäßrigen Methylamin-Lösung getropft, anschließend die Temperatur nach Entfernen des Kältebades auf Raumtemperatur ansteigen gelassen, 30 Minuten nachgerührt, die erhaltene Suspension im Wasserstrahlvakuum eingeengt und der Rückstand mit 300 ml Wasser verrührt. Der Feststoff wurde abgesaugt und zur Entfernung von geringen Mengen an Ausgangsmaterial und an in der 2-Position substituiertem Produkt an Kieselgel (Laufmittel Toluol/EtOH 15:1) chromatografiert.

Man erhielt an Produkt 22 g (89 % der theoretischen Ausbeute) Fp.: 72 - 3 ° C.

Beispiel 3

Herstellung von 2,4-Dichlorthiazol-sulfonsäure-N-isopropylamid

Analog zu Beispiel 2 wurde das 2,4-Dichlorthiazol-sulfonsäure-N-isopropylamid durch Umsetzung von 2,4-Dichlorthiazol-sulfonsäurechlorid aus Beispiel 1 mit 65 %iger wäßriger Isopropylamin-Lösung hergestellt.

Nach Umkristallisation aus Petrolether/Aceton 6:1 erhielt man an Produkt 26,7 g (97 % der theoretischen Ausbeute), Fp.: 99-100 ° C.

Beispiel 4

Herstellung von 2,4-Dichlorthiazol-sulfonsäure-N,N-dimethylamid

Analog zu Beispiel 2 wurde das 2,4-Dichlorthiazol-sulfonsäure-N,N-dimethylamid durch Umsetzung von 2,4-Dichlorthiazol-sulfonsäurechlorid aus Beispiel 1 mit 45 %iger Dimethylamin-Lösung hergestellt. Nach Umkristallisieren aus Petrolether/Aceton 6:1 erhielt man an Produkt 21,7 g (83 % der theoretischen Ausbeute), Fp.: 89-90 ° C.

Beispiel 4A

Herstellung von 2,4-Dichlorthiazol-sulfonsäure-N-cyclopropylamid

Analog zu Beispiel 2 wurde das 2,4-Dichlorthiazolsulfonsäure-N-cyclopropylamiddurch Umsetzung von 2,4-Dichlorthiazol-sulfonsäurechlorid aus Beispiel 1 mit wasserfreiem Cyclopropylamin hergestellt. Nach Umkristallisation aus Toluol erhielt man 24,4 g Produkt (89,2% der theoretischen Ausbeute), Fp.: 90-91 ° C.

Beispiel 4B

Herstellung von 2,4-Dichlorthiazol-sulfonsäure-N-allylamid

Analog zu Beispiel 2 wurde das 2,4-Dichlorthiazol-sulfonsäure-N-allylamid durch Umsetzung von 2,4-Dichlorthiazolsulfonsäurechlorid aus Beispiel 1 mit wasserfreiem Allylamin hergestellt. Man erhielt nach Umkristallisieren aus Petrolether/Aceton (6 : 1) 7,6 g Produkt (28 % der theoretischen Ausbeute), Fp.: 80-81 ° C.

Beispiel 5

Herstellung von 2-N,N-Dimethylamino-4-chlorthiazol-sulfonsäure-N-methylamid

Zu 24,7 g (0,1 Mol) 2,4-Dichlorthiazol-sulfonsäuremethylamid aus Beispiel 2 in 250 ml THF wurden unter Eisbadkühlung in ca. 20 Min. 33,2 g einer 45 %igen wäßrigen Dimethylamin-Lösung getropft. Anschließend wurde auf Raumtemperatur erwärmen gelassen, 30 Min. nachgerührt, im Wasserstrahlvakuum eingeengt und der Rückstand mit 250 ml Wasser verrührt. Der Feststoff wurde abgesaugt, getrocknet und aus Toluol umkristallisiert. Man erhielt an farblosen Kristallen 23 g (90 %), Fp.: 139 ° C.

Beispiel 6

Herstellung von 2-N-Phenylamino-4-chlorthiazol-sulfonsäure-N-methylamid

Analog zu Beispiel 5 wurde das 2-N-Phenylamino-4-chlorthiazol-sulfonsäure-N-methylamid durch Umsetzung von 2,4-Dichlorthiazol-sulfonsäuremethylamid aus Beispiel 2 mit Anilin hergestellt. Nach Chromatographie an Kieselgel mit Methylenchlorid als Laufmittel erhielt man in einer Ausbeute von 15 % das Produkt mit einem Schmelzpunkt von 157-8 ° C.

Beispiel 7

Herstellung von 2-N-Isopropylamino-4-chlorthiazol-sulfonsäure-N-methylamid

Analog zu Beispiel 5 wurde das 2-N-Isopropylamino-4-chlorthiazol-sulfonsäure-N-methylamid durch Umsetzung von 2,4-Dichlorthiazol-sulfonsäuremethylamid aus Beispiel 2 mit einer 65 %igen wäßrigen Isopropylamin-Lösung umgesetzt. Nach Umkristallisieren aus Toluol erhielt man 89 % Ausbeute an Produkt vom Fp. 135 ° C.

Beispiel 8

Herstellung von 2-N,N-Dimethylamino-4-chlorthiazol-sulfonsäure-N-isopropylamid

Analog zu Beispiel 5 wurde das 2-N,N-Dimethylamino-4-chlorthiazol-sulfonsäure-N-isopropylamid durch Umsetzung von 2,4-Dichlorthiazol-sulfonsäure-N-isopropylamid aus Beispiel 3 mit 45 %iger wäßriger Dimethylamin-Lösung hergestellt. Nach Umkristallisieren aus Toluol erhielt man in einer Ausbeute von 74 % das Produkt mit einem Fp. von 138 ° C.

Beispiel 9

Herstellung von 2-N-Methylamino-4-chlorthiazol-sulfonsäure-N-isopropylamid

Analog zu Beispiel 5 wurde das 2-N-Methylamino-4-chlorthiazol-sulfonsäure-N-isopropylamid durch Umsetzung von 2,4-Dichlorthiazol-sulfonsäure-N-isopropylamid aus Beispiel 3 mit 30 %iger wäßriger Methylamin-Lösung hergestellt. Nach Umkristallisation aus Toluol erhielt man in einer Ausbeute von 78 % der theoretischen Ausbeute das Produkt mit einem Fp. von 187-8 ° C.

Beispiel 10

Herstellung von 2-N-Methylamino-4-chlorthiazol-sulfonsäure-N,N-dimethylamid

Analog zu Beispiel 5 wurde das 2-N-Methylamino-4-chlorthiazol-sulfonsäure-N,N-dimethylamid durch Umsetzung von 2,4-Dichlorthiazol-sulfonsäure-N,N-dimethylamid aus Beispiel 4 mit 30 %iger wäßriger Methylamin-Lösung hergestellt. Nach Umkristallisation aus Toluol erhielt man an Produkt 88 % der theoretischen Ausbeute mit einem Fp. von 194-195 ° C.

Beispiel 11

Herstellung von 2-N-Isopropylamino-4-chlorthiazol-sulfonsäure-N,N-dimethylamid

Analog zu Beispiel 5 wurde das 2-N-Isopropylamino-4-chlorthiazol-sulfonsäure-N,N-dimethylamid durch Umsetzung von 2,4-Dichlorthiazol-sulfonsäure-N,N-dimethylamid aus Beispiel 4 mit einer 65 %igen wäßrigen Isopropylamin-Lösung hergestellt. Man erhielt ein öliges Produkt it einem Brechungsindex $n_D^{20}$ von 1.4563 in einer Ausbeute von 92 % der theoretischen Ausbeute.

Beispiel 12

Herstellung von 2-N-Phenylamino-4-chlorthiazol-sulfonsäure-N,N-dimethylamid

Analog zu Beispiel 5 wurde das 2-N-Phenylamino-4-chlorthiazol-sulfonsäure-N,N-dimethylamid durch Umsetzung von 2,4-Dichlorthiazol-sulfonsäure-N,N-dimethylamid aus Beispiel 4 mit Anilin hergestellt. Nach Umkristallisation aus Methylenchlorid erhielt man in einer Ausbeute von 47 % der theoretischen Ausbeute und einem Fp. von 162 ° C das Produkt.

Beispiel 12A

Herstellung von 2-N,N-Dimethylamino-4-chlor-thiazol-sulfonsäure-N-cyclopropylamid

Analog zu Beispiel 5 wurde das 2-N,N-Dimethylamino-4-chlorthiazolsulfonsäure-N-cyclopropylamid durch Umsetzung von 2,4-Dichlorthiazolsulfonsäure-N-cyclopropylamid aus Beispiel 4A mit einer 45%igen

wäßrigen Dimethylamin-Lösung hergestellt. Nach Umkristallisation aus Toluol erhielt man 26,8 g farblose Kristalle (96,5 % der theoretischen Ausbeute), Fp.: 146-147 ° C.

Beispiel 12B

Herstellung von 2-N,N-Dimethylamino-4-chlor-thiazol-sulfonsäure-N-allylamid

Analog zu Beispiel 5 wurde das 2-N,N-Dimethylamino-4-chlorthiazolsulfonsäure-N-allylamid durch Umsetzung von 2,4-Dichlorthiazolsulfonsäure-N-allylamid aus Beispiel 4B mit einer 45%igen wäßrigen Dimethylamin-Lösung hergestellt. Nach Chromatografie an Kieselgel mit Toluol/ Ethanol (5 : 1) als Laufmittel erhielt man 15,7 g Produkt (56 % der theoretischen Ausbeute).

[Referenzbeispiel für die nachfolgenden Beispiele 13, 14, 15 und 15A:

Herstellung von 2-N-(3-Methoxy-n-propylamino)-4-chlorthiazol-sulfonsäure-N-(3-methoxy-n-propyl)-amid

Zu einer Lösung von 25,25 g (0,1 Mol) 2,4-Dichlorthiazol-sulfonsäurechlorid aus Beispiel 1 in 600 ml THF wurden bei Raumtemperatur 40 g 3-Methoxy-n-propylamin getropft und anschließend 30 Min. bei Raumtemperatur nachgerührt. Nach dem Einengen im Wasserstrahlvakuum wurde mit 500 ml Wasser verrührt, die wäßrige Phase zweimal mit Methylenchlorid extrahiert und die vereinigten organischen Phasen über Magnesiumsulfat getrocknet. Nach Entfernen des Lösungsmittels im Wasserstrahlvakuum erhielt man 31,3 g eines braunen Öls, das an Kieselgel mit Toluol/Ethanol (5:1) als Laufmittel chromatographiert wurde. Man erhielt an Produkt 29,3 g (79 %), Fp.: 55 ° C.]

Beispiel 13

Herstellung von 2-N-Ethylamino-4-chlorthiazol-sulfonsäure-N-ethylamid

Analog zum Referenzbeispiel wurde das 2-N-Ethylamino-4-chlorthiazol-sulfonsäure-N-ethylamid durch Umsetzung von 2,4-Di-chlorthiazol-sulfonsäurechlorid aus Beispiel 1 mit Ethylamin hergestellt. Nach Umkristallisation aus Toluol erhielt man in 63 %iger Ausbeute das Produkt mit einem Fp. von 104 ° C.

Beispiel 14

Herstellung von 2-N-(2,2-Dimethyl-propylamino)-4-chlorthiazol-sulfonsäure-N-(2,2-dimethylpropyl)-amid

Analog zum Referenzbeispiel wurde das 2-N-(2,2-Dimethyl-propylamino)-4-chlorthiazol-sulfonsäure-N-(2,2-dimethylpropyl)-amid durch Umsetzung von 2,4-Dichlorthiazolsulfonsäurechlorid aus Beispiel 1 mit 2,2-Dimethylpropylamin hergestellt. Nach Umkristallisation aus Toluol erhielt man in einer Ausbeute von 67 % und mit einem Fp. von 157 ° C das Produkt.

Beispiel 15

Herstellung von 2-N-Methylamino-4-chlorthiazol-sulfonsäure-N-methylamid

Analog zum Referenzbeispiel wurde das 2-N-Methylamino-4-chlorthiazol-sulfonsäure-N-methylamid durch Umsetzung von 2,4-Dichlorthiazol-sulfonsäurechlorid aus Beispiel 1 mit 30 %iger wäßriger Methylamin-Lösung hergestellt. Nach Umkristallisation aus Diisopropylether erhielt man in einer Ausbeute von 66 % das Produkt mit einem Fp. von 200 ° C.

Beispiel 15A

Herstellung von 2-N-Cyclopropylamino-4-chlorthiazolsulfonsäure-N-cyclopropylamid

Analog zum Referenzbeispiel wurde das 2-N-Cyclopropylamino-4-chlorthiazolsulfonsäure-N-cyclopropylamid durch Umsetzung von 2,4-Dichlorthiazol-sulfonsäurechlorid aus Beispiel 1 mit Cyclopropylamin hergestellt. Nach Chromatographie an Kieselgel mit Toluol/Ethanol (5 : 1) als Laufmittel erhielt man 15,0 g Produkt (51 % der theoretischen Ausbeute), Fp.: 176-177 ° C.

Beispiel 16

Herstellung von 2-(N-Dichlorfluormethylsulfenyl)-methylamino-4-chlorthiazol-sulfonsäure-dimethylamid

Zu einer Lösung von 25,55 g (0,1 Mol) 2-Methylamino-4-chlorthiazol-sulfonsäure-dimethylamid aus Beispiel 10 in 100 ml Methylenchlorid, der 10,5 g Triethylamin zugesetzt waren, wurde bei Raumtemperatur in ca. 20 Min. eine Lösung von 16,95 g (0,1 Mol) Dichlorfluormethylsulfenchlorid in 100 ml $CH_2C1_2$ zugetropft. Es wurde 2 h bei Raumtemperatur nachgerührt, anschließend zweimal mit je 100 ml Wasser gewaschen, die organische Phase über Magnesiumsulfat getrocknet und am Rotationsverdampfer eingeengt. Man erhielt 36 g eines gelben Feststoffs, der über Kieselgel mit Methylenchlorid als Laufmittel chromatographiert wurde. Man erhielt an Produkt 30,3 g farblose Kristalle (78 %), Fp.: 118°C.

Beispiele 17-23

Analog zu Beispiel 16 wurden die in der Tabelle 1 aufgeführten, monosulfenylierten Produkte hergestellt:

Tabelle 1

| Beispiel Nr. | Ausgangsverbindung aus Beispiel-Nr. | Reaktionsprodukt 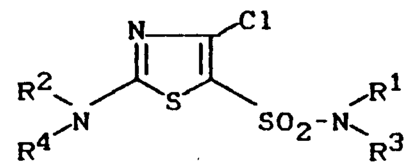 | | | | Fp. (°C) | Ausbeute (%) |
|---|---|---|---|---|---|---|---|
| | | $R^1$ | $R^3$ | $R^2$ | $R^4$ | | |
| 17 | 11 | $CH_3$ | $CH_3$ | $(CH_3)_2CH$ | $SCFCl_2$ | 112 | 37 |
| 18 | 12 | $CH_3$ | $CH_3$ | $C_6H_5$ | $SCFCl_2$ | 132 | 48 |
| 19 | 5 | $CH_3$ | $SCFCl_2$ | $CH_3$ | $CH_3$ | 115 | 91 |
| 20 | 8 | $(CH_3)_2CH$ | $SCFCl_2$ | $CH_3$ | $CH_3$ | 116 | 32 |
| 21 | 6 | $CH_3$ | $SCFCl_2$ | $C_6H_5$ | H | 152 | 61 |
| 22 | 12A | Cyclo-propyl | $SCFCl_2$ | $CH_3$ | $CH_3$ | 122 | 95 |
| 23 | 12B | $CH_2=CH-CH_2$ | $SCFCl_2$ | $CH_3$ | $CH_3$ | Öl | 28 |

Beispiel 24

Herstellung von 2-(N-Dichlorfluormethylsulfenyl)-methylamino-4-chlorthiazol-sulfonsäure-(N-dichlorfluormethylsulfenyl-N-methvl)-amid

Zu einer Lösung von 24,15 g (0,1 Mol) 2-Methylamino-4-chlorthiazol-sulfonsäure-methylamid aus Beispiel 15 in 300 ml Toluol wurden 37,3 g (0,22 Mol) Dichlorfluormethylsulfenchlorid und anschließend 22,22 g (0,22 Mol) Triethylamin bei Raumtemperatur zugetropft. Man ließ 1 h bei Raumtemperatur, dann 1 h bei 40 ° C nachrühren. Es wurden anschließend 200 ml Wasser zugesetzt, die organische Phase über $MgSO_4$ getrocknet und am Rotationsverdampfer eingeengt. Nach chromatographischer Filtration des Rohproduktes an Kieselgel mit Methylenchlorid als Laufmittel und Einengen der Lösung erhielt man das Produkt als schwach gelbes, zähes Öl mit einem Brechungsindex $n_D^{20}$ von 1,5825. Die Ausbeute betrug 40,75 g (51 % der theoretischen Ausbeute.

Beispiele 25-29

Analog zu Beispiel 24 wurden die in der Tabelle 2 aufgeführten, zweifach sulfenylierten Produkte erhalten:

Tabelle 2

| Beispiel Nr. | Ausgangsverbindung aus Beispiel-Nr. | Reaktionsprodukt 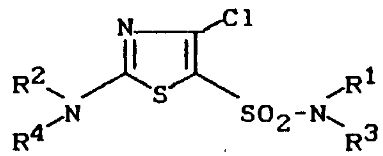 | | | | Physikalische Daten | Ausbeute (%) |
|---|---|---|---|---|---|---|---|
| | | $R^1$ | $R^3$ | $R^2$ | $R^4$ | | |
| 25 | 13 | $C_2H_5$ | $SCFCl_2$ | $C_2H_5$ | $SCFCl_2$ | $^{20}_{D}$ : 1.5695 | 78 |
| 26 | 7 | $CH_3$ | $SCFCl_2$ | $CH_3 \rangle CH$ $CH_3$ | $SCFCl_2$ | Fp.: 82° C | 85 |
| 27 | 9 | $CH_3 \rangle CH$ $CH_3$ | $SCFCl_2$ | $CH_3$ | $SCFCl_2$ | Fp.: 91° C | 77 |
| 28 | 14 | $(CH_3)_3C-CH_2$ | $SCFCl_2$ | $(CH_3)_3C-CH_2$ | $SCFCl_2$ | Fp.: 118° C | 88 |
| 29 | 15A | Cyclopropyl | $SCFCl_2$ | Cyclopropyl | $SCFCl_2$ | Fp.: 101° C | 31 |

Anwendungsbeispiele

Beispiel a)

Venturia-Test (Apfel) / protektiv

| | |
|---|---|
| Lösungsmittel: | 4,7 Gewichtsteile Aceton |
| Emulgator: | 0,3 Gewichtsteile Alkyl-aryl-polyglykolether |

Zur Herstellung einer zweckmäßigen Wirkstoffzubereitung vermischte man 1 Gewichtsteil Wirkstoff mit den angegebenen Mengen Lösungsmittel und Emulgator und verdünnte das Konzentrat mit Wasser auf die gewünschte Konzentration.

Zur Prüfung auf protektive Wirksamkeit bespritzte man junge Pflanzen mit der Wirkstoffzubereitung bis zur Tropfnässe. Nach Antrocknen des Spritzbelages wurden die Pflanzen mit einer wäßrigen Konidiensuspension des Apfelschorferregers (Venturia inaequalis) inokuliert und verblieben dann 1 Tag bei 20°C und 100 % relativer Luftfeuchtigkeit in einer Inkubationskabine.

Die Pflanzen wurden dann im Gewächshaus bei 20°C und einer relativen Luftfeuchtigkeit von ca. 70 % aufgestellt.

12 Tage nach der Inokulation erfolgte die Auswertung.

Eine deutliche Überlegenheit in der Wirksamkeit gegenüber dem Stand der Technik zeigten in diesem Test z.B. die Verbindungen aus den in Tabelle 3 angegebenen Herstellungsbeispielen:

Tabelle 3

Venturia-Test (Apfel) / protektiv

| Wirkstoff | | | Wirkungsgrad in % der unbehandelten Kontrolle bei einer Wirkstoffkonzentration von 5 ppm |
|---|---|---|---|
| gemäß Stand der Technik | | | |
| | 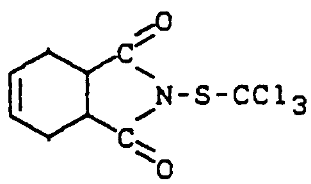 | Captan | 63 |
| erfindungsgemäß | | | |
| Herstell-Beispiel-Nr. | | | |
| 16 | 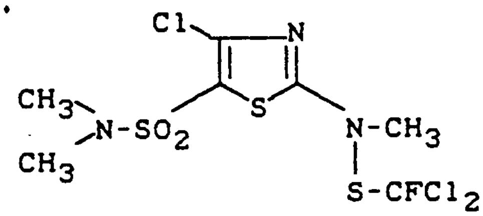 | | 89 |
| 17 | 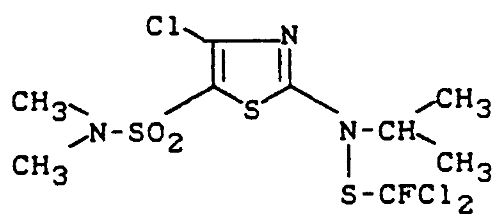 | | 70 |
| 19 | 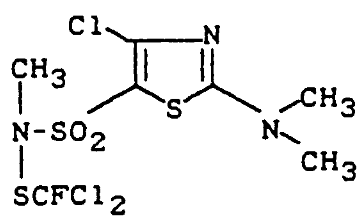 | | 82 |

Tabelle 3 (Fortsetzung)

Venturia-Test (Apfel) / protektiv

| Wirkstoff | | Wirkungsgrad in % der unbehandelten Kontrolle bei einer Wirkstoffkonzentration von 5 ppm |
|---|---|---|
| Herstell-Beispiel-Nr. | | |
| 20 | 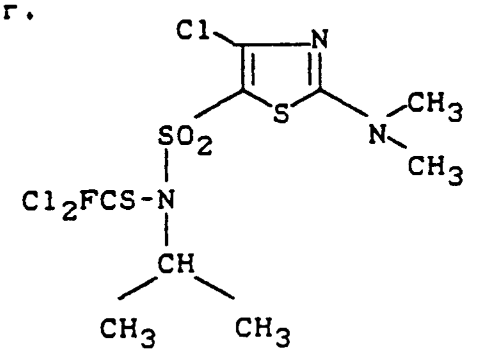 | 88 |
| 24 | 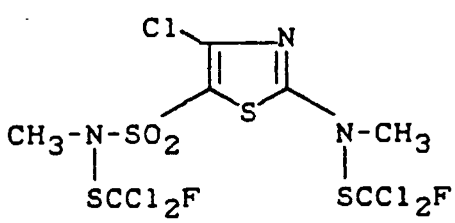 | 86 |

Beispiel b)

Botrytis-Test (Buschbohne) / protektiv

| | |
|---|---|
| Lösungsmittel: | 4,7 Gewichtsteile Aceton |
| Emulgator: | 0,3 Gewichtsteile Alkyl-aryl-polyglykolether |

Zur Herstellung einer zweckmäßigen Wirkstoffzubereitung vermischte man 1 Gewichtsteil Wirkstoff mit den angegebenen Mengen Lösungsmittel und Emulgator und verdünnte das Konzentrat mit Wasser auf die gewünschte Konzentration.

Zur Prüfung auf protektive Wirksamkeit bespritzte man junge Pflanzen mit der Wirkstoffzubereitung bis zur Tropfnässe. Nach Antrocknen des Spritzbelages wurden auf jedes Blatt 2 kleine mit Botrytis cinerea bewachsene Agarstückchen aufgelegt. Die inokulierten Pflanzen wurden in einer abgedunkelten, feuchten Kammer bei 20 °C aufgestellt. 3 Tage nach der Inokulation wurde die Größe der Befallsflecken auf den Blättern ausgewertet.

Eine deutliche Überlegenheit in Wirksamkeit gegenüber dem Stand der Technik zeigten in diesem Test z.B. die Verbindungen aus den in Tabelle 4 angegebenen Herstellungsbeispielen:

Tabelle 4

Botrytis-Test (Buschbohne) /protektiv

| Wirkstoff | | Wirkungsgrad in % der unbehandelten Kontrolle bei einer Wirkstoffkonzentration von 100 ppm |
|---|---|---|
| Herstell-Beispiel-Nr. | | |
| 16 | 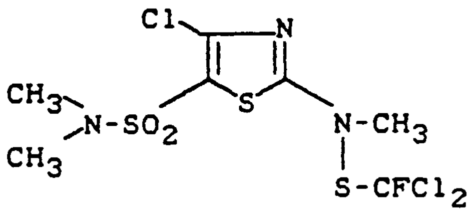 | 90 |
| 17 | 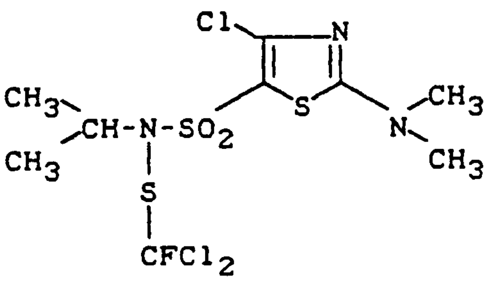 | 95 |
| 19 | 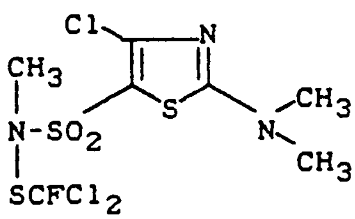 | 96 |

Tabelle 4 (Fortsetzung)

Botrytis-Test (Buschbohne) /protektiv

| Wirkstoff | | Wirkungsgrad in % der unbehandelten Kontrolle bei einer Wirkstoffkonzentration von 100 ppm |
|---|---|---|
| Herstell-Beispiel-Nr. | | |
| 20 | 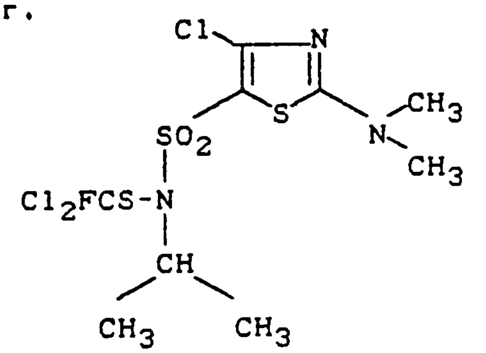 | 82 |
| 24 | 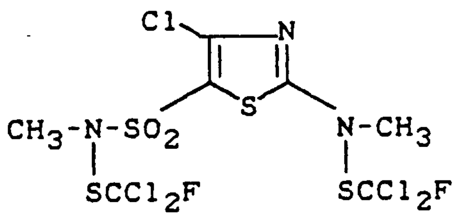 | 100 |

## Patentansprüche

1. N-sulfenylierte 2-Amino-4-chlor-thiazol-sulfonamide der Formel (I)

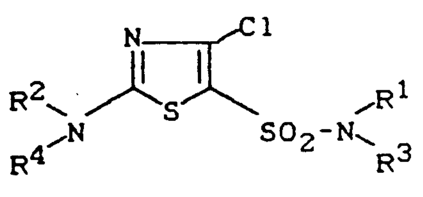

(I),

worin

$R^1$ und $R^2$ unabhängig voneinander geradkettiges oder verzweigtes $C_1$-$C_6$-Alkyl, Allyl, Crotyl, Propargyl, $C_3$-$C_6$-Cycloalkyl, Phenyl, Benzyl, Phenylethyl, 2-Furyl, 2-, 3- oder 4-Pyridyl, 2- oder 4-Pyrimidyl oder 2-Morpholinyl bedeuten und

$R^3$ und $R^4$ unabhängig voneinander für Wasserstoff, geradkettiges oder verzweigtes $C_1$-$C_6$-Alkyl, $C_3$-$C_6$-Cycloalkyl, Phenyl, Benzyl oder Phenylethyl oder für den Dichlorhalogenomethylsulfenylrest $-S-CCl_2X$, in welchem X Fluor, Chlor oder Brom bedeutet, stehen,

wobei mindestens einer der Reste $R^3$ und $R^4$ für den Rest $-S-CCl_2X$ steht.

2. N-sulfenylierte 2-Amino-4-chlor-thiazol-sulfonamide der Formel (I) gemäß Anspruch 1, dadurch gekennzeichnet, daß darin

   $R^1$ und $R^2$ unabhängig voneinander geradkettiges oder verzweigtes $C_1$-$C_6$-Alkyl, Allyl, Crotyl, Propargyl, $C_3$-$C_6$-Cycloalkyl, Phenyl oder Benzyl bedeuten und

   $R^3$ und $R^4$ unabhängig voneinander für Wasserstoff, geradkettiges oder verzweigtes $C_1$-$C_6$-Alkyl oder den Dichlorhalogenomethylsulfenylrest -S-$CCl_2$X, in welchem X Fluor, Chlor oder Brom bedeutet, stehen,

   wobei mindestens einer der Reste $R^3$ und $R^4$ für den Rest -S-$CCl_2$X steht.

3. Verfahren zur Herstellung von N-sulfenylierten 2-Amino-4-chlor-thiazol-sulfonamiden der Formel (I) gemäß Anspruch 1,
   dadurch gekennzeichnet, daß man in einer ersten Stufe 2,4-Dichlor-thiazol mit Chlorsulfonsäure zu 2,4-Dichlor-thiazol-sulfonsäurechlorid der Formel (II)

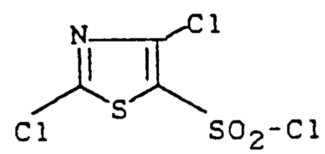

(II)

   umsetzt, in einer zweiten Stufe das 2,4-Dichlorthiazol-sulfonsäurechlorid mit Aminen der Formeln (III) oder (II)

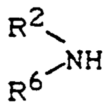

(III) bzw.

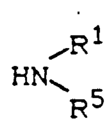

(IV),

   worin

   $R^1$ und $R^2$ die angegebene Bedeutung haben und

   $R^5$ und $R^6$ unabhängig voneinander für Wasserstoff, $C_1$-$C_6$-Alkyl, $C_3$-$C_6$-Cycloalkyl, Phenyl oder Benzyl stehen,

   wobei mindestens einer der Reste $R^5$ und $R^6$ Wasserstoff bedeutet,
   zu 2-Amino-4-chlor-thiazol-sulfonamiden der Formel (V)

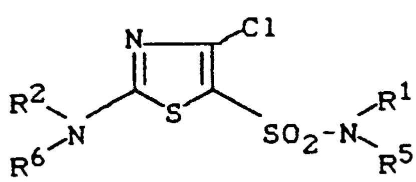

(V)

   umsetzt, worin

   $R^1$, $R^2$, $R^5$ und $R^6$ die angegebene Bedeutung haben,

   und in einer dritten Stufe die 2-Amino-4-chlorthiazol-sulfonamide mit Dichlorhalogenomethylsulfenylchloriden der Formel (VI)

   Cl-S-$CCl_2$X (VI),

   worin

   X Fluor, Chlor oder Brom bedeutet,

   umsetzt.

4. 2,4-Dichlor-thiazol-sulfonsäurechlorid der Formel (II)

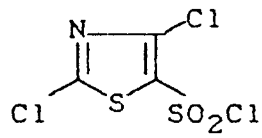

(II) .

5. 2-Amino-4-chlor-thiazol-sulfonamide der Formel (V)

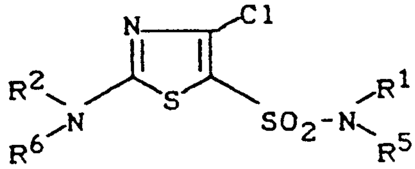

(V) ,

worin

$R^1$ und $R^2$ unabhängig voneinander geradkettiges oder verzweigtes $C_1$-$C_6$-Alkyl, Allyl, Crotyl, Propargyl, $C_3$-$C_6$-Cycloalkyl, Phenyl, Benzyl, Phenylethyl, 2-Furyl, 2-, 3-oder 4-Pyridyl, 2- oder 4-Pyrimidyl oder 2-Morpholinyl bedeuten und

$R^5$ und $R^6$ unabhängig voneinander für Wasserstoff, geradkettiges oder verzweigtes $C_1$-$C_6$-Alkyl, $C_3$-$C_6$-Cycloalkyl, Phenyl, Benzyl oder Phenylethyl stehen,

wobei mindestens einer der Reste $R^5$ und $R^6$ Wasserstoff bedeutet.

6. 2-Amino-4-chlor-thiazol-sulfonamide der Formel (V) gemäß Anspruch 5, dadurch gekennzeichnet, daß darin

$R^1$ und $R^2$ unabhängig voneinander geradkettiges oder verzweigtes $C_1$-$C_6$-Alkyl, Allyl, Crotyl, Propargyl, $C_3$-$C_6$-Cycloalkyl, Phenyl oder Benzyl bedeuten und

$R^5$ und $R^6$ unabhängig voneinander für Wasserstoff oder geradkettiges oder verzweigtes $C_1$-$C_6$-Alkyl stehen,

wobei mindestens einer der Reste $R^5$ und $R^6$ Wasserstoff bedeutet.

7. Verwendung der Stoffe nach Anspruch 1 als Pflanzenschutzmittel.

**Claims**

1. N-Sulphenylated 2-amino-4-chloro-thiazole-sulphonamides of the formula (I)

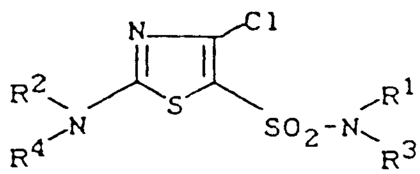

(I),

wherein

$R^1$ and $R^2$ independently of one another denote straight-chain or branched $C_1$-$C_6$-alkyl, allyl, crotyl, propargyl, $C_3$-$C_6$-cycloalkyl, phenyl, benzyl, phenylethyl, 2-furyl, 2-, 3- or 4-pyridyl, 2- or 4-pyrimidyl or 2-morpholinyl and

$R^3$ and $R^4$ independently of one another represent hydrogen, straight-chain or branched $C_1$-$C_6$-alkyl, $C_3$-$C_6$-cycloalkyl, phenyl, benzyl or phenylethyl or the dichlorohalogenomethylsulphenyl radical -S-$CCl_2X$ in which X denotes fluorine, chlorine or bromine, with at least one of the radicals $R^3$ and $R^4$ representing the radical -S-$CCl_2X$.

2. N-Sulphenylated 2-amino-4-chloro-thiazole-sulphonamides of the formula (I) according to Claim 1, characterised in that in said formula

$R^1$ and $R^2$ independently of one another denote straight-chain or branched $C_1$-$C_6$-alkyl, allyl, crotyl, propargyl, $C_3$-$C_6$-cycloalkyl, phenyl or benzyl and
$R^3$ and $R^4$ independently of one another represent hydrogen, straight-chain or branched $C_1$-$C_6$-alkyl or the dichlorohalogenomethylsulphenyl radical -S-$CCl_2X$ in which X denotes fluorine, chlorine or bromine, with at least one of the radicals $R^3$ and $R^4$ representing the radical -S-$CCl_2X$.

**3.** Process for the preparation of N-sulphenylated 2-amino-4-chloro-thiazole-sulphonamides of the formula (I) according to Claim 1, characterised in that, in a first step, 2,4-dichloro-thiazole is reacted with chlorosulphonic acid to give 2,4-dichloro-thiazole-sulphonyl chloride of the formula (II)

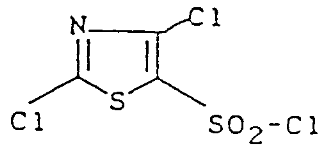

(II)

in a second step, the 2,4-dichloro-thiazole-sulphonyl chloride is reacted with amines of the formula (III) or (IV)

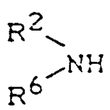

(III) or

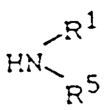

(IV),

wherein
$R^1$ and $R^2$ have the abovementioned meaning and
$R^5$ and $R^6$ independently of one another represent hydrogen, $C_1$-$C_6$-alkyl, $C_3$-$C_6$-cycloalkyl, phenyl or benzyl,
with at least one of the radicals $R^5$ and $R^6$ denoting hydrogen,
to give 2-amino-4-chloro-thiazole-sulphonamides of the formula (V)

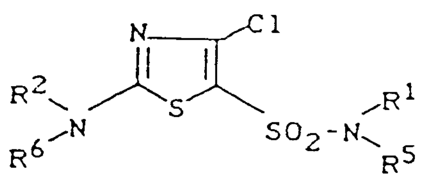

(V)

wherein
$R^1$, $R^2$, $R^5$ and $R^6$ have the abovementioned meaning, and, in a third step, the 2-amino-4-chloro-thiazole-sulphonamides are reacted with dichlorohalogeno-methylsulphenyl chlorides of the formula (VI)

Cl-S-$CCl_2X$ (VI)

wherein
X denotes fluorine, chlorine or bromine.

**4.** 2,4-Dichloro-thiazole-sulphonyl chloride of the formula (II)

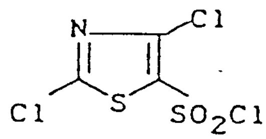

(II).

**5.** 2-Ainino-4-chloro-thiazole-sulphonamides of the formula (V)

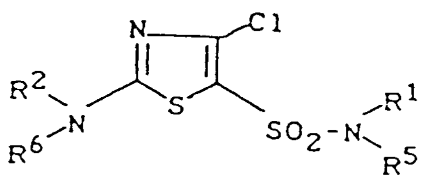

(V),

wherein
R' and $R^2$ independently of one another denote straight-chain or branched $C_1$-$C_6$-alkyl, allyl, crotyl, propargyl, $C_3$-$C_6$-cycloalkyl, phenyl, benzyl, phenylethyl, 2-furyl, 2-, 3- or 4-pyridyl, 2- or 4-pyrimidyl or 2-morpholinyl and $R^5$ and $R^6$ independently of one another represent hydrogen, straight-chain or branched $C_1$-$C_6$-alkyl, $C_3$-$C_6$-cycloalkyl, phenyl, benzyl or phenylethyl,
with at least one of the radicals $R^5$ and $R^6$ denoting hydrogen.

**6.** 2-Amino-4-chloro-thiazole-sulphonamides of the formula (V) according to Claim 5, characterised in that in said formula
$R^1$ and $R^2$ independently of one another denote straight-chain or branched $C_1$-$C_6$-alkyl, allyl, crotyl, propargyl, $C_3$-$C_6$-cycloalkyl, phenyl or benzyl and
$R^5$ and $R^6$ independently of one another represent hydrogen or straight-chain or branched $C_1$-$C_6$-alkyl, with at least one of the radicals $R^5$ and $R^6$ denoting hydrogen.

**7.** Use of substances according to Claim 1 as plant protection agents.

**Revendications**

**1.** 2-amino-4-chlorothiazole-sulfonamides N-sulfénylés de formule I

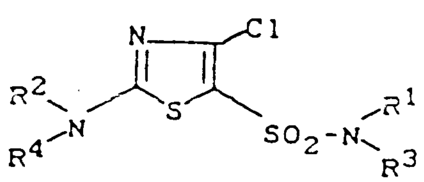

(I),

dans laquelle

| | |
|---|---|
| $R^1$ et $R^2$ | représentent chacun, indépendamment l'un de l'autre, un groupe alkyle en $C_1$-$C_6$ à chaîne droite ou ramifiée, un groupe allyle, crotonyle, propargyle, cycloalkyle en $C_3$-$C_6$, phényle, benzyle, phényléthyle, 2-furyle, 2-, 3- ou 4-pyridyle, 2- ou 4-pyrimidyle ou 2-morpholinyle, et |
| $R^3$ et $R^4$ | représentent chacun indépendamment l'un de l'autre, l'hydrogène, un groupe alkyle en $C_1$-$C_6$ à chaîne droite ou ramifiée, un groupe cycloalkyle en $C_3$-$C_6$, phényle, benzyle ou phényléthyle ou le groupe dichlorohalogénométhylsulfényle $-S-CCl_2X$ dans lequel X représente le fluor, le chlore ou le brome, |

l'un au moins des symboles $R^3$ et $R^4$ représentant le groupe $-S-CCl_2X$.

**2.** 2-amino-4-chlorothiazole-sulfonamides N-sulfénylés de formule I de la revendication 1, caractérisés en ce que :

$R^1$ et $R^2$ représentent chacun, indépendamment l'un de l'autre, un groupe alkyle en $C_1$-$C_6$ à chaîne droite ou ramifiée, un groupe allyle, crotonyle, propargyle, cycloalkyle en $C_3$-$C_6$, phényle ou benzyle, et

$R^3$ et $R^4$ représentent chacun, indépendamment l'un de l'autre, l'hydrogène, un groupe alkyle en $C_1$-$C_6$ à chaîne droite ou ramifiée ou le groupe dichlorohalogénométhylsulfényle -S-$CCl_2X$ dans lequel X représente le fluor, le chlore ou le brome,

l'un au moins des symboles $R^3$ et $R^4$ représentant le groupe -S-$CCl_2X$.

**3.** Procédé de préparation des 2-amino-4-chlorothiazole-sulfonamides N-sulfénylés de formule I de la revendication 1, caractérisé en ce que, dans un premier stade opératoire, on fait réagir le 2,4-dichlorothiazole avec l'acide chlorosulfonique, la réaction donnant le chlorure de l'acide 2,4-dichlorothiazole-sulfonique de formule II

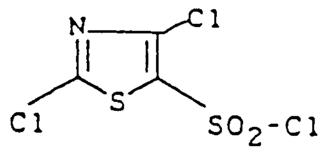

(II)

qu'on fait réagir dans un deuxième stade avec des amines de formules III ou IV

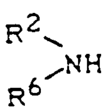

(III) ou

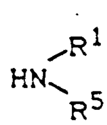

(IV),

dans lesquelles

$R^1$ et $R^2$ ont les significations indiquées ci-dessus, et

$R^5$ et $R^6$ représentent chacun, indépendamment l'un de l'autre, l'hydrogène, un groupe alkyle en $C_1$-$C_6$, cycloalkyle en $C_3$-$C_6$, phényle ou benzyle,

l'un au moins des symboles $R^5$ et $R^6$ représentant l'hydrogène,

la réaction donnant les 2-amino-4-chlorothiazole-sulfonamides de formule V

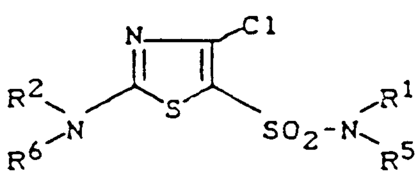

(V)

dans laquelle $R^1$, $R^2$, $R^5$ et $R^6$ ont les significations indiquées ci-dessus, qu'on fait réagir dans un troisième stade opératoire avec des chlorures de dichlorohalogénométhylsulfényle de formule VI

Cl-S-$CCl_2X$ (VI),

dans laquelle X représente le fluor, le chlore ou le brome.

**4.** Le chlorure de l'acide 2,4-dichlorothiazole-sulfonique de formule II

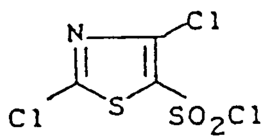

(II) .

5. 2-amino-4-chlorothiazole-sulfonamides de formule V

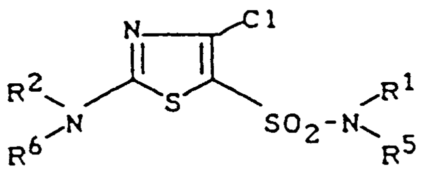

(V) ,

dans laquelle

$R^1$ et $R^2$ représentent chacun, indépendamment l'un de l'autre, un groupe alkyle en $C_1$-$C_6$ à chaîne droite ou ramifiée, un groupe allyle, crotonyle, propargyle, cyaloalkyle en $C_3$-$C_6$, phényle, benzyle, phényléthyle, 2-furyle, 2-, 3- ou 4-pyridyle, 2- ou 4-pyrimidyle ou 2-morpholinyle, et

$R^5$ et $R^6$ représentent chacun, indépendamment l'un de l'autre, l'hydrogène, un groupe alkyle en $C_1$-$C_6$ à chaîne droite ou ramifiée, un groupe cycloalkyle en $C_3$-$C_6$, phényle, benzyle ou phénéthyle,

l'un au moins des symboles $R^5$ et $R^6$ représentant l'hydrogène.

6. 2-amino-4-chlorothiazole-sulfonamides de formule V de la revendication 5, caractérisés en ce que :

$R^1$ et $R^2$ représentent chacun, indépendamment l'un de l'autre, un groupe alkyle en $C_1$-$C_6$ à chaîne droite ou ramifiée, un groupe allyle, crotonyle, propargyle, cycloalkyle en $C_3$-$C_6$, phényle ou benzyle, et

$R^5$ et $R^6$ représentent chacun, indépendamment l'un de l'autre, l'hydrogène ou un groupe alkyle en $C_1$-$C_6$ à chaîne droite ou ramifiée,

l'un au moins des symboles $R^5$ et $R^6$ représentant l'hydrogène.

7. Utilisation des composés selon la revendication 1 en tant que produits phytosanitaires.